Europäisches Patentamt

European Patent Office

Office européen des brevets

(11) EP 1 146 128 A1

(19)

(12) **EUROPEAN PATENT APPLICATION**

(43) Date of publication:
**17.10.2001 Bulletin 2001/42**

(51) Int Cl.[7]: **C12Q 1/34**, C12Q 1/68,
G01N 33/573

(21) Application number: **00830285.3**

(22) Date of filing: **14.04.2000**

(84) Designated Contracting States:
**AT BE CH CY DE DK ES FI FR GB GR IE IT LI LU
MC NL PT SE**
Designated Extension States:
**AL LT LV MK RO SI**

(71) Applicant: **Università degli Studi di Roma " Tor
Vergata"**
**00173 Roma (IT)**

(72) Inventors:
• **Maccarrone, Mauro**
**67100 l'Aquila (IT)**
• **Valensise, Herbert**
**00136 Roma (IT)**

• **Finazzi, Agro', Alessandro**
**00198 Roma (IT)**

(74) Representative: **Bazzichelli, Alfredo et al**
**c/o Società Italiana Brevetti S.p.A.**
**Piazza di Pietra, 39**
**00186 Roma (IT)**

Remarks:
The sequence listing, which is published as annex
to the application documents, was filed after the date
of filing. The applicant has declared that it does not
include matter which goes beyond the content of the
application as filed.

(54) **Method and kit for the early diagnosis of miscarriage in human beings**

(57) The present invention relates to a method for the diagnosis of miscarriage in female human beings based on the detection of the presence of the enzyme anandamide hydrolase (FAAH) active in blood cells and in particular in lymphocytes, and a diagnosis kit useful in the performance of said method.

FIG. 1B

**Description**

Field of the Invention

The present invention relates to the diagnosis of spontaneous abortion in human beings.

State of the art

Spontaneous abortion is characterized by embryonic death by heart failure, with no evidence of other morphological or functional anomalies (Kline et al. 1989; Sozio e Ness 1998; Goldstein in *Clin* Obstet Gynecol 1994; Redline et al. 1999). It takes place due to causes and as a consequence of mechanisms that are totally unknown.
It constitutes the most common adverse outcome of pregnancy, entailing non negligible social costs, and patients' pain and suffering.
Early markers of miscarriage have long been sought in order to meet the demand for a human population screening. (Goldstein in *Clin Obstet Gynecol* 1994; Redline et al. 1999). In fact, since women experiencing spontaneous abortion are also totally healthy subjects, not evidencing any clinical sign anticipating an adverse outcome of the pregnancy, a predictive diagnostic method possibly applicable to a large population would be particularly desirable.
To date, spontaneous abortion has been associated to the variations of the levels of some molecules physiologically present in pregnant women, like increased perivillous fibrin, increased levels of serum androgen or urine beta-core fragment, variation of chorionic gonadotropin levels (Sozio and Ness 1998; Goldstein in *Obstet Gynecol* 1994; Okon et al. 1998; Cole et al. 1997; Kovalevskaya et al. 1999).
Concerning the first three markers, the detection of alterations in the respective levels is not always predictive of the spontaneous abortion, that, moreover, in this case is due to specific clinical situations of the subject. Further, the detection of such alterations is unsuitable for human population screenings, as it is based on non-routine techniques, that are therefore difficult, lengthy and expensive.
As for the beta-subunit of human chorionic gonadotropin, even though at present its dosage is clinically used as a marker of the first trimester of gestation, (Goldstein in *Obstet Gynecol* 1994), it is well-known that its levels do not always correlate with an early embryonic death, and even its quantitation has been recently questioned. (Kovalevskaya et al. 1999).
Genetic analysis of epoxide hydrolase polymorphism and/or microsatellite mutations has also been associated with spontaneous miscarriage (Wang et al. 1998; Spandidos et al. 1998). It proved to be unsuitable for a screening procedure of a large population, since it requires highly specialized personnel and structures, besides being time-consuming and expensive.
Recently, the enzyme anandamide hydrolase (hereinafter referred to also as FAAH) has been isolated and characterized for its ability to hydrolize biologically active endocannabinoids, in particular the endocannabinoid anandamide (arachidonoylethanolamide) on the human and animal neural cells (Maccarrone et al. 1998).
Endocannabinoids are endogenous compounds known to be physiologically produced and to exert their function on the human and animal neural cells (Di Marzo et al. 1998; Stella et al. 1997). The denomination of endocannabinoids is specifically due to the ability of such compounds to act as endogenous ligands for both brain ($CB_1$) and peripheral ($CB_2$) cannabinoid receptors (Pertwee 1997). In particular, the ability of the endocannabinoids has been demonstrated to mimic, following the binding thereof with human and animal $CB_1$ and $CB_2$ receptors, the psychotropic, hypnotic, tranquillizing effects on memory and attention that are known in the art to be a consequence of the administration of cannabinoids (Devane et al. 1992; Calignano et al. 1998; Stella et al. 1997).
The effects on human fertility, notoriously associated to the administration of cannabinoids to pregnant women, are the blocking of the blastocyst division and hatching and the induction of a retarded embryo development (Das et al. 1995; Yang et al. 1996; Schmid et al. 1997; Paria et al. 1998).
Similar adverse effects on pregnancy were reported for the endocannabinoids as well, and specifically for the anandamide (Das et al. 1995; Yang et al. 1996; Schmid et al. 1997; Paria et al. in 1998). However, the cytotoxic action of the (endo)cannabinoids has been reported exclusively on animal models, and only following the external administration thereof. As for human beings, drug use by the mother is known to have adverse effects on pregnancy, though to date evidence on the causes of this phenomenon have yet to be reported (Kline et al. 1989; Ness et al. 1999).
However, in human beings as well as in animal models, no data whatsoever has been reported, that demonstrate or even merely hinting at an interference of the endocannabinoids physiologically present in the body with the normal pregnancy development.
Moreover, it has to be pointed out that the adverse effects on pregnancy found in mouse following anandamide administration do not emerge as spontaneous abortions, but rather as a blocking of the blastocyst hatching, a stopping of the cell division of the blastocyst itself at the tetracellular stage and of the embryonal growth and development (Das et al. 1995; Yang et al. 1996; Schmid et al. 1997; Paria et al. 1998).

Thus, as the art is not aware of any connection existing between endocannabinoids and spontaneous abortion, all the more so the art is aware of no data, neither in human beings nor in animal models, demonstrating, or even merely hinting at, an involvement of the anandamide hydrolase enzyme in spontaneous abortion.

In fact, in the animal models where the effect of the anandamide on the embryo implantation was studied, the FAAH presence was reported merely in uterine tissue and in the embryo (Paria et al. 1999).

In humans, where the physiological action of the anandamide is known only in neural cells, FAAH presence in brain fragment was reported (Maccarrone et al. 1998). Moreover, FAAH presence in platelets (Maccarrone et al. in *FEBS Lett* 1999) and in human mastocytes (Maccarrone et al. 2000) was also reported, yet without it being associated to any physiological action.

Summary of the Invention

The subject matter of the present invention is a method for the early diagnosis of miscarriage in female human beings comprising the following steps:

    a. Detecting the presence of the enzyme anandamide hydrolase (FAAH) active in the blood cells of said female human being, said detection yielding a detected value;
    b. comparing said detected value and a threshold value therebetween, said threshold value being predetermined in order to be indicative of the probability of miscarriage in human beings.

A first advantage of the method of the present invention is that it allows an early diagnosis of miscarriage, already at 6 weeks of gestation.

A second advantage of the method of the present invention is that it can be carried out even on non-pregnant women, in order to obtain a diagnostic indication of the probability to suffer miscarriage in case of pregnancy by women having low FAAH levels in blood cells.

A further advantage of the method of the present invention lies in the fact that it merely requires a blood sampling in order to be performed.

A further advantage of the method of the present invention lies in the fact that anyhow, in the various embodiments thereof, it has a remarkable quickness of execution, requiring from 2 to 8 hours to be completed.

A further advantage of the method of the present invention is provided by its cost-effectiveness, as the costs of the performance thereof are those typical of any other blood analysis.

A further advantage of the method of the present invention is the detection sensitivity of the adopted techniques, enabling to carry out analyses on a reduced amount of blood sample (down to 0.5 ml). Such detection can be carried out on any blood cell, and in particular on lymphocytes and platelets.

In particular, the detection of which at step a, can be performed by assaying the enzymatic activity of the hydrolase in the cell, or analyzing the expression of the anandamide hydrolase in the blood cells. The activity assay can be performed with any technique able to quantitate enzymatic activity among those known in the art, like, e.g. the spectrophotometry, the polarography, the chromatography, in particular the high performance liquid chromatography (HPLC), the luminescence and the isotopic methods, all described in Wilson and Walker 1995. In this respect, a measuring carried out with the HPLC technique, in all the variants thereof known to the art is particularly advantageous. Specific advantages are associated to the use of the inverted-phase HPLC technique (hereinafter also referred to as I-HPLC) described in Maccarrone et al. in *Anal. Biochem.* 1999.

This technique entails the advantage of enabling the steps of promoting the reaction between the enzyme and the radioactive substrate and of extracting the product in organic solvents to be performed in a single vessel. This advantage is due to the fact that the enzyme-substrate reaction is performed using a 20 µg/200 µl ratio between the protein amount and the enzymatic reaction mixture volume, and to the fact that the first extracting step is performed with a 1:4 volume ratio between the reaction mixture and the extraction mixture. Casewise, also a C18 (5 µm, 30x3 mm i.d.) column is used, with the further advantage of allowing a greater quickness of execution of the method (HPLC analysis of the product in a few minutes). The steps of said process are reported in detail in Maccarrone et al. in *Anal. Biochem.* 1999.

Therefore, with respect to other HPLC processes, the I-HPLC allows to reduce the number of sample manipulations and the time required therefor, in order to allow a remarkable number of measurements in a few minutes. This process further entails the great advantage of combining the accuracy and the precision of the HPLC methods to the sensitivity of the isotopic methods.

In fact, this embodiment has the advantage of a high sensitivity of the detection, in particular allowing the detection on an enzyme amount equal to a few µg of total cell proteins, with a measuring sensitivity reaching the femtomoles of product per minute.

Concerning the method of the present invention, in which the detection of which at step a. is performed by analizing the expression of the anandamide hydrolase in the blood cells, this analysis can be performed by measuring the presence of the protein in the analyzed cells, i.e., by measuring the presence of the mRNA coding therefor in said cells. In this latter instance, any one technique among those known in the art allowing the detection of the FAAH-specific mRNA concentration in the analyzed cells can be used. For instance, the Northern blot and the reverse transcriptase polymerase chain reaction (RT-PCR) can be used (Wilson and Walker 1995).

The RT-PCR processes, in all the variants thereof known to the art aimed at the amplification of a specific sequence, are particularly advantageous in the method of the present invention due to the detection sensitivity thereof. Thereamong, a RT-PCR process specifically developed for FAAH measuring and particularly advantageous in the method as to the present invention is the one described in detail in Maccarrone et al. 1998. In particular, said process is characterized by the use of the following amplification parameters: 2 min at 45 °C, 45 sec at 95 °C, 30 sec at 55 °C and 30 sec at 60 °C, with a linear amplification performed for 20 cycles, using as primers the oligonucleotides having a sequence reported in the annexed sequence listing from SEQ ID N: 1 to SEQ ID NO: 4.

Quantitative data was obtained measuring the amount of amplified product yielded by RT-PCR. This measuring can be performed with all the methods known to the art to be useful for this purpose. When the amplification is carried out with radioactive material, the quantitation can be performed by measuring the radioactivity corresponding to the amplification bands with liquid scintillation counting, as described, e.g., in Maccarrone et al. 1998.

When, instead, the analysis of the anandamide hydrolase expression is performed by measuring the presence of the protein in the analyzed cells, such measurement can be performed by any one technique allowing to detect the presence of proteins in a cell. For instance, techniques like, e.g., Western blot and enzyme-linked immunosorbent assay (ELISA) can be adopted. (cfr., e.g., Wilson and Walker 1995).
Accordingly, the embodiment of the method in which the detection is not performed on cell homogenate, but rather on intracellular material obtained by lysing the cells directly in the medium in which the binding reaction between the enzyme and the specific antibody, an ELISA technique that will hereinafter be referred to as cellular ELISA is particularly advantageous. Actually, this technique can be applied to the quantitation of any protein, hence not exclusively in the method of the present invention, according to the modifications that can be effected by those skilled in the art according to what is known to the art.

In fact, performing step a. with this technique reduces the -number of steps to be performed on the sample, making the assay quicker and increasing the sample yield. This entails that a smaller amount of blood is required in order to obtain an amount of cells sufficient for a reliable FAAH detection. Therefore, in this preferred embodiment, the method of the present invention can be performed on a very low cell population (down to $0.4 \times 10^6$; to this regard, see also infra).

Moreover, in light of the techniques commonly adopted for the cell purification from the blood sample (5 ml blood can yield $7.5 - 10 \times 10^6$ lymphocytes), this entails the further advantages of:

- performing a greater number of tests on the same sample, and
- using samples containing a minute amount of blood,

    with respect to other ELISA techniques, without altering the statistical significance of the results.

In fact, using the purification techniques known to the art, from no more than 1 ml peripheral blood 1.5 to $2 \times 10^6$ lymphocytes can be extracted, a cell amount sufficient in this embodiment to perform triplicate analyses.

Moreover, the cellular ELISA method can also be performed by means of a robot in a particularly easy way, with further advantages in terms of costs and of a reduced need of skilled operators. This is especially relevant, in view of a spreading of the technique in hospital facilities as well.

The method of the present invention, in which the FAAH detection is performed by cellular ELISA, can be performed on a medium spacious enough to contain an amount of reactants, denominated wells (in this case functioning as reaction vessels) sufficient to the reaction.

In a particularly preferred embodiment, the detection by ELISA, i.e., cellular ELISA, can be performed using the following compositions:

    coating composition (C.B. composition) comprising a chemical reactant able to adhere the cell proteins onto the well walls (preferably 500 mM $Na_2CO_3$) and an agent inhibiting the growth of microorganisms, in particular of bacteria (preferably 0.2% $NaN_3$) at the reaction temperature;
    washing composition (W.B. composition) comprising a physiological buffered solution, preferably with a phosphate buffer (PBS), and a detergent, preferably of polyoxyethylenic type, in particular 0.01% Tween-20;
    blocking composition (B.B. composition) comprising the washing composition (W.B.) and a saturating protein, preferably gelatine, said saturating protein being able to saturate the aspecific sites of the reaction vessel, said saturating protein being comprised in an amount sufficing to attain, due to the addition of said B.B. composition in said reaction vessel, the saturation of said aspecific sites of said vessel;

Diluting composition (D.B. composition) comprising the washing buffer (W.B.) and the saturating protein comprised in the blocking composition, said saturating protein being comprised in said diluting composition in an amount equal to one third of the amount of said protein in the blocking composition;

by the following steps in sequence:

- adding a different cellular suspension volume equal to 1/10 of the final volume to a vessel;
- adding to said vessel bidistilled water or equivalent solution to a volume equal to 8/10 of the final volume, and incubating it for ≤ 5min at room temperature;
- adding to said vessel a volume equal to 1/10 of the final volume of the C.B. composition, and incubating all for ≤ 2 hours at room temperature;
- collecting the content of said vessel and adding an amount of B.B. composition sufficient to fill said vessel, and incubating all for ≤ 2 hours at room temperature.
- washing said vessel at least 4 times with the W.B. composition, and once with the D.B. composition;
- adding to said vessel a volume equal to one half of the final volume of the D.B. composition and a primary antibody (i.e., a FAAH-specific antibody) diluted in order to attain an effective bond to the target enzyme, and incubating all for ≤ 1.5 hours at room temperature;
- washing said well at least 4 times with the W.B. composition and once with the D.B. composition;
- adding to each well a volume equal to one half of the final volume of a secondary antibody (i.e., a primary antibody-specific antibody) suitably diluted in the D.B. composition in order to obtain a measurable bond between said secondary antibody and the primary antibody, and incubating for ≤1.5 hours at room temperature;
- washing said well at least 5 times with one volume of the W.B. composition and adding the substrate of the secondary antibody-conjugated enzyme, in an amount sufficient to allow the measuring of the reaction;
- incubating, under conditions adequate to allow the completion of the reaction between the secondary antibody-conjugated enzyme and the related substrate.

Then, upon completion of the final step, the absorbance values can be read at the appropriate wavelength.

Monoclonal, as well as polyclonal, antibodies can be used in the procedure; in particular, the primary antibodies should be specific human anti-FAAH antibodies, the secondary antibodies should be specific for the primary antibodies. In particular, as primary antibodies, human FAAH-specific polyclonal antibodies produced in the rabbit against an ovalbumin-conjugated peptide and corresponding to the conserved FAAH sequence reported in the sequence listing as SEQ ID NO: 5 (Giang et al. 1997) were successfully used. However, any other polyclonal or monoclonal specific antibody can be used as well, in the case of the monoclonal antibody with the further advantage of an improved detection sensitivity. These antibodies can be produced by a person skilled in the art according to processes currently known to the art.

In general, from the detection of enzymatically active anandamide hydrolase performed according to any one of the above-disclosed embodiments, a value is obtained that can be indicative of the protein activity (in particular by the preferable processes HPLC and I-HPLC), of the protein concentration (in particular by the processes of the preferred embodiments ELISA or Cellular ELISA), or of the concentration of mRNA coding for the enzyme (in particular by the process of the preferred embodiment RT-PCR).

Therefore, for each of these embodiments such value has to be compared to a threshold value predetermined in order to be indicative of the probability of a spontaneous abortion.

In particular, this value can be predetermined measuring the value in a statistically significant population and assessing, e.g. by a clinical study, the value ranges associated to the occurrence of a spontaneous abortion, and to a normal pregnancy. In light of this assessment, comparing the two value ranges therebetween it will then be possible to set a threshold value such that the values therebelow be indicative of the probability of spontaneous abortion, whereas the values above such threshold are indicative of a probable normal gestation.

In light of a study conducted on a cohort of 50 women according to modalities detailed below in the next paragraph, concerning the method in the embodiment in which the detection at step a. is performed by ELISA, i.e. cellular ELISA, such value was assessed to be equal to $0.15 \pm 0.02$ absorbance units at 405 nm.

For the HPLC process, the threshold was equal to $95 \pm 10$ pmoles of product per minute per mg of protein.

Concerning the RT-PCR process, and in particular the process in which the amplified product quantitation is performed by measuring the radioactivity of the amplified bands, the threshold value was equal to $7500 \pm 800$ cpm.

A further subject matter of the present invention is a kit for the early diagnosis of miscarriage in female human beings, comprising

a coating composition (C.B. composition) comprising a chemical reactant able to adhere the cell proteins onto the well walls and an agent inhibiting the growth of microorganisms, in particular bacteria, at the reaction temperature;
a primary antibody composition comprising a primary antibody, said primary antibody being an anandamide hydrolase- (FAAH) specific antibody, and a compatible carrier,

said compositions being useful according to the above-disclosed method wherein the detection of the enzyme expression is performed by ELISA, i.e., cellular ELISA.

Concerning the coating composition, the reactant able to increase the protein adhesion and the agent inhibiting the bacterial growth can be any reactant and agent compatible therebetween among those known to the art to be useful in an ELISA reaction, at the concentrations and under the conditions known in the art to be compatible to the functionality thereof.

In a preferred embodiment, the composition comprises 500 mM $Na_2CO_3$ as reactant, 0.2% $NaN_3$ as agent, all at a pH 9.6.

Concerning the primary antibody composition, the FAAH-specific primary antibody can be a monoclonal, as well as a polyclonal antibody, provided it is human FAAH-specific. In particular, human FAAH-specific polyclonal antibodies produced in the rabbit against an ovalbumin-conjugated peptide, corresponding to the conserved FAAH sequence reported in the sequence listing as SEQ ID NO: 5 can be used (Giang et al. 1997). However, any other polyclonal or monoclonal specific antibody can be profitably used, with the advantage of an improved detection sensitivity for the latter. These antibodies can be produced by a person skilled in the art according to the current procedures known in the art. The compatible carrier of the primary antibody composition is any one carrier among those known in the art to be useful for the formulation of solutions containing antibodies.

Moreover, the kit of the present invention can comprise a further composition denominated washing composition (W.B.), comprising a physiological buffered solution, preferably with a phosphate buffer (PBS), containing a detergent, preferably of polyoxyethylenic type, and preferably 0.01% Tween-20.

Moreover, a blocking composition comprising the washing composition as above-defined and a saturating protein can further be comprised, said saturating protein being a protein able to saturate the aspecific sites of the reaction vessel, said saturating protein being comprised in said blocking composition in a sufficient amount in order to attain, following the addition of said blocking composition in said reaction vessel, the saturation of the aspecific sites of said reaction vessel, and

a diluting composition comprising the washing composition as above defined and the saturating protein comprised in the blocking composition, preferably gelatine, said saturating protein being comprised in said diluting composition in an amount equal to one third of the amount of said saturating protein in the blocking composition, whereas said blocking composition and said diluting composition are useful in the method of the present invention in the embodiment in which the FAAH detection is performed by ELISA or cellular ELISA.

In a preferred embodiment, the kit comprises, alternatively to or in addition to said blocking and diluting compositions, the compositions required to prepare them at the moment of use.

Therefore, concerning the blocking composition, in such embodiment the washing composition and a protein composition (defined as blocking composition), comprising in a compatible carrier the saturating protein as above defined, in an amount such that due to the addition of said protein blocking composition to an adequate amount of the washing composition the blocking composition as above-defined is obtained, are comprised.

Therefore, concerning the diluting composition, in such embodiment the washing composition and a protein composition (defined as diluting composition), comprising the saturating protein as above-defined, in a compatible carrier, in an amount such that due to the addition of said protein diluting composition to an adequate amount of washing composition the diluting composition as above-defined is obtained, are comprised.

Hence, the advantage of not having to preserve said compositions, that instead are freshly-prepared.

In another embodiment, the kit already comprises the empty vessels for the blocking and for the diluting composition, i.e. with such an amount of washing composition in each that it suffices to addition the blocking and diluting protein compositions as above-defined in order to obtain the blocking and diluting compositions.

A vessel comprising bidistilled water or equivalent solution to be used in the method of the present invention in the embodiment using the Cellular ELISA for the detection of which at step a. can also be comprised in the kit of the present invention.

Furthermore, a secondary antibody composition comprising, in a compatible carrier, a secondary antibody specific for the primary antibody contained in the primary antibody composition can also be comprised, said secondary antibody being conjugated to a quantitatively detectable molecule, and a detecting composition comprising, in a compatible carrier, a molecule that yields a measurable product interacting with the detectable molecule of the secondary antibody composition.

Such compatible carrier can be any carrier among those known in the art to be useful in this type of composition. Concerning the detectable antibody-conjugated molecule and the molecule interacting therewith that define the detection system, besides several enzyme-substrate systems known in the art, in which the enzyme is bound to the secondary antibody, other systems known to the art, like, e.g., the biotin/avidine one can be used. In the preferred embodiment, the secondary antibody-conjugated molecule is the alkaline phosphatase enzyme, bound to the secondary antibody, and the molecule contained in the detecting composition is the hep-nitrophenylphosphate substrate.

All the compositions of the kit of the present invention can be comprised in such an amount as to be used in one, or preferably 10, typical ELISA plates (each having 96 wells of 240 µl each) (concentrations denominated 1X and 10 X respectively). The ELISA plate itself can be included in the kit.

All these embodiments of the kit of the present invention have the advantage of allowing an easier performance of the method of the present invention in the embodiment in which the enzyme detection is performed by ELISA i.e., cellular ELISA. In particular, the embodiments comprising an ever-increasing number of compositions entail the advantage of an easier and quicker performance of the method of the present invention in the form wherein the detection of which at step a. is performed by ELISA, i.e., cellular ELISA.

Description of the Figures

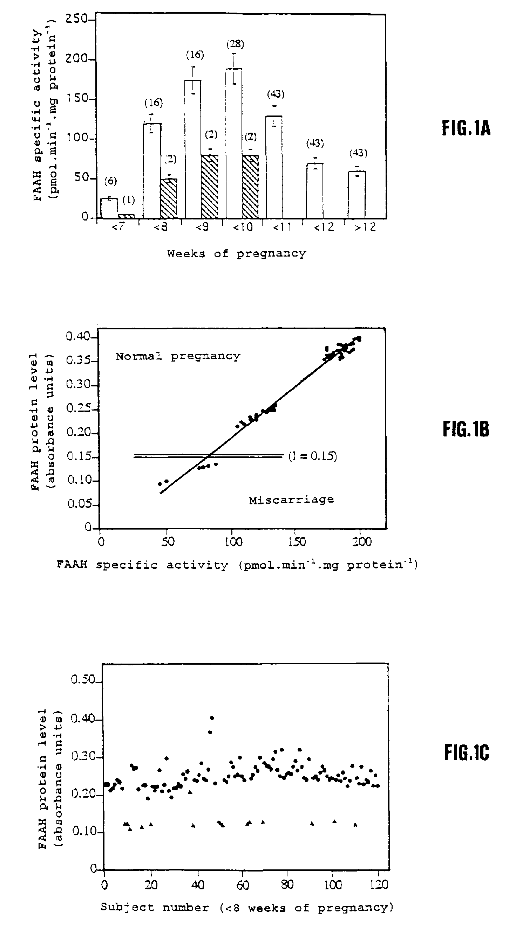

Figure 1 shows the anandamide hydrolase (FAAH) levels in subjects with normal pregnancy and in subjects who suffered a spontaneous abortion.

(A) Results of study A, where FAAH activity was measured in lymphocytes isolated from 50 healthy women and associated a *posteriori* with normal pregnancy (empty bars) or miscarriage (hatched bars), are shown in panel A. The mean values (± S.D.) of measurements performed (each in triplicate) on the number of subjects shown in parenthesis are reported.

(B) The correlation between FAAH activity and FAAH protein levels in the same pregnant subjects reported in panel A is shown in panel B. The threshold level (1) between the abortion group and the normal group is shown in brackets. In particular, the single measurements (each mean of a triplicate) performed on <8- to <10-week pregnant subjects are reported.

(C) Results of study B, where FAAH protein was measured in lymphocytes isolated from 120 healthy women, and associated a *posteriori* with normal pregnancy (circles) or miscarriage (triangles), are shown in panel C. In particular, the results of single measurings (each mean of a triplicate), performed on <8-week pregnant women, are shown.

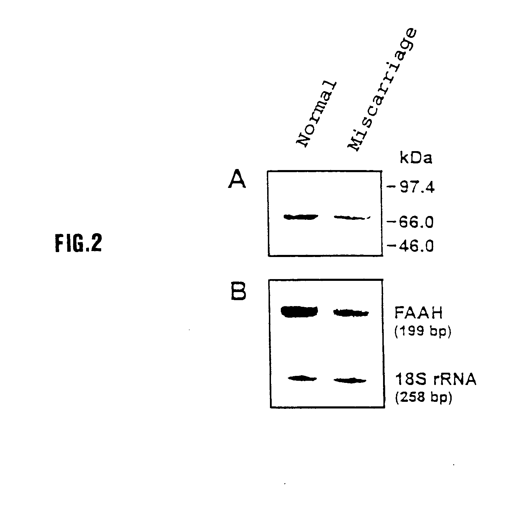

Figure 2 shows the anandamide hydrolase (FAAH) levels in subjects with normal pregnancy and in subjects who suffered a spontaneous abortion.

(A) The outcome of a Western blot analysis performed on proteins of human lymphocytes (20 µg/lane) from <8-week-pregnant women is shown in Panel A. Molecular mass markers, phosphorylase b (97.4 kDa), bovine serum albumin (66.0 kDa) and ovalbumin (46.0 kDa) are shown on the right-hand side.

(B) The outcome of a RT-PCR of FAAH mRNA (50 ng/lane) and 18S rRNA (0.2 ng/lane), isolated from the same subjects as in panel A, is shown in Panel B. The product size (bp) from RT- PCR is reported in brackets.

Figure 3 shows the linearity ratio between the absorbance values measured through the performance of the method and the number of examined cells.

Figure 4 shows the linearity ratio between the absorbance values measured through the performance of the method and the amount of examined cell proteins.

Detailed Description of the Invention

The method of the present invention was elaborated in light of the observation that low levels of the enzyme anandamide hydrolase in the blood cells, and in particular in lymphocytes and platelets, are predictive of spontaneous abortion.

In particular, this observation followed a 2-stage study.

A first stage (study A) was aimed at finding the possible association between the levels of FAAH in blood cells, in particular in peripheral lymphocytes, and spontaneous abortion in humans.

Fifty low-risk patients selected according to their clinical history were enrolled for study A. In particular, patients with chronic diseases (diabetes, hypertension, Lupus eryhtematosus systemicus), with a known history of recurrent spontaneous abortions, with uterine anomalies, or under chronic ongoing treatments (corticosteroids, low dose aspirin), with any sign of vaginal bleeding, abdominal pain or any other abnormal sign were excluded from enrollment.

Among those 50 patients, 7 were <7-week, 12 were <8-week, 2 were <9-week, 14 were <10-week and 15 were <11-week-pregnant, and were all observed up to 13 weeks with repeated ultrasound (US) and blood tests. Then, patients were examined at 22 and 32 weeks of gestation.

Gestational age was assessed on the basis of the date of the last normal menstrual period, confirmed through the first Crown Rump Length (CRL) measurement.

No difference in age, gravidity, parity and body-mass index could be observed between women who miscarried and those who did not. Both groups showed the same ratios of previous spontaneous abortions. The anatomical and embryonic structures (such as gestational sac and yolk sac), the embryo and the cardiac rate were all within normality at the first ultrasound scan (Goldstein in *Obstet Gynecol* 1994).

Lymphocytes were purified from peripheral blood by gradient centrifugation, using the density separation medium Lymphoprep (Nycomed Pharma) (Maccarrone et al. 1997).

The activity of FAAH (arachidonoylethanolamide amidohydrolase, EC 3.5.1.4) in human lymphocyte homogenates (20 µg/test) was determined by measuring the release of [1-$^{14}$C]arachidonic acid from [1-$^{14}$C]anandamide (NEN DuPont de Nemours, 52 mCi/mmol), using reversed phase high performance liquid cromatography as reported in Maccarrone et al. 1998.

Spontaneous (missed) abortion was defined as the absence of cardiac activity in the embryo, assessed by ultrasonography. All the ultrasound examinations were performed using a 6.5 MHz transvaginal probe and a 3.5 MHz transabdominal probe (AU4 IDEA Esaote, Ansaldo).

In light of the measuring performed in all subjects in various stages of pregnancy, a lower FAAH activity in lymphocytes of women who experienced spontaneous abortion, compared to those who did not (see figure 1A) could be observed. The difference in FAAH activity between the group of 43 subjects with normal pregnancy (mean value = 169.60 $\pm$ 30.20 pmol.min$^{-1}$.mg protein$^{-1}$) and the group of 7 women who aborted (mean value 60.43 $\pm$ 29.34 pmol. min$^{-1}$.mg protein$^{-1}$) was statistically significant (P<0.0001 by the Mann-Whitney test). Embryonic death occurred approximately 10 days later, i.e. at 8 to 11 weeks for the subjects <7- to <10-week pregnant, respectively. Kinetic analysis of FAAH activity at <8 weeks of pregnancy showed an apparent Michaelis-Menten constant (Km) of 8 $\pm$ 1 µM in subjects with normal gestation and those who miscarried, whereas the apparent maximum velocity (Vmax) values were 187 $\pm$ 20 and 75 $\pm$ 8 pmol.min$^{-1}$.mg protein$^{-1}$, respectively. On the other hand, the bell-shaped curve found for FAAH activity in normal subjects is frequently observed with biological indicators of the first trimester of pregnancy, such as the beta-subunit of human chorionic gonadotropin (Goldstein in *Obstet Gynecol* 1994).

At this stage of the study, assessing whether the decreased FAAH activity in lymphocytes of women who experienced a spontaneous abortion could be due to a reduced expression of the enzyme was deemed of interest. Therefore, FAAH activity at 8 weeks of gestation was correlated with FAAH (by Western blot and ELISA) and the FAAH-coding mRNA (by RT-PCR) contents in the same samples.

Western blotting of FAAH was performed on human lymphocyte homogenates (20 µg/sample), electrophoresed on a 12% polyacrylamide gel with SDS, and electroblotted on 0.45 µm nitrocellulose filters, using specific FAAH polyclonal antibodies (Primm), diluted 1:200 (16), and then goat anti-rabbit polyclonal antibodies (Bio-Rad Laboratories), conjugated with alkaline phosphatase and diluted 1:2000 (Maccarrone et al. 1998). This Western blotting has identified FAAH as a single immunoreactive band of about 67 kDa (figure 2A).

In light of these analyses, a lower FAAH band intensity could be observed in subjects experiencing spontaneous abortion compared to those who did not (figure 2A). The same antibodies were used to quantitate the FAAH protein content by ELISA. Wells were coated with human lymphocyte homogenates (20 µg/well), which were then reacted with anti-FAAH polyclonal antibodies (diluted 1:300), as first antibody, and with goat anti-rabbit polyclonal antibodies, conjugated with alkaline phosphatase (Bio-Rad Laboratories), diluted 1:2000 (Maccarrone et al. 1998), as second antibody. Color development of the alkaline phosphatase reaction was measured at 405 nm, using p-nitrophenyl phosphate as substrate. The ELISA test was linear in the range 0-50 µg/well of cell homogenate and its specificity for FAAH was validated by antigen competition experiments (Maccarrone et al. 1998), as the purified enzyme is not available to make calibration curves.

The same measurement was performed following the addition of a hypotonic solution like bidistilled water in the wells of the lymphocyte-containing Falcon plates, and a 5 min incubation, in order to allow a complete cytolysis, confirmed by a cytophluorometric analysis that evidenced an 100% cytolysis. This enabled a direct use of the isolated cells, with no need of any additional homogenating step (Cellular ELISA). The adhering of the cell proteins to the well, required for the subsequent antibody reaction, is ensured by the use of a coating buffer (C.B.) 10-fold more concentrated than usual These two measures (osmotic shock with bidistilled water and more concentrated C.B.) allowed the performance of all the dosage steps directly on the cells, rather than on cell homogenates, in particular automatically by a robot.

These experiments evidenced a significant linear correlation (R>0.99) found between FAAH specific activity and FAAH protein levels quantitated by ELISA in <8- to <10- week-pregnant subjects (figure 1B). This indicates that the decreased activity of the FAAH in the lymphocytes of women experiencing spontaneous abortion is due to a reduced expression of the gene coding for the enzyme.

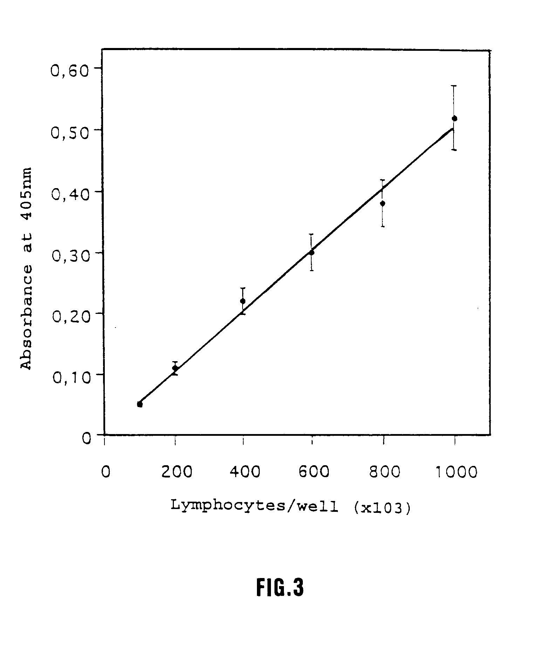

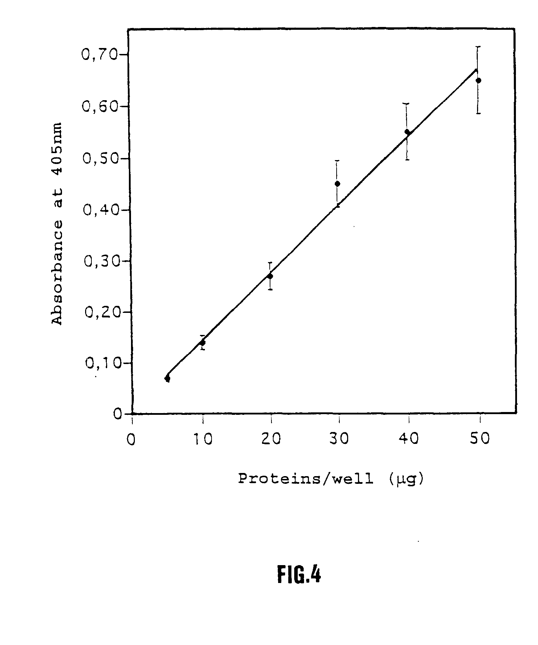

In particular, it has to be stressed that the dosage method of the FAAH levels in peripheral lymphocytes evidenced a very satisfactory linearity between the absorbance values at 405 nm (corrected for the aspecific absorbance of the bovine serum albumin and the amount of sample under test, expressed as number of cells as well as protein amount. The maximum values of the linearity range were $1.0 \times 10^6$ lymphocytes (Figure 3) and 50 μg proteins/well (Figure 4). On the other hand, the minimum measurable amounts (with a > 3:1 signal/noise ratio) were $0.1 \times 10^6$ lymphocytes or 5 μg proteins/well, indicating the high sensitivity of the test. The linear interpolation of typical experimental points, depicted in Figures 4 and 5, generates two straight lines, described by the equations:

$$Y = 0.006 + 0.0005X \ (R = 0.996) \qquad \text{(Figure 4)}$$

$$Y = 0.013 + 0.0132X \ (R = 0.995) \qquad \text{(Figure 5)}$$

Concerning the subsequent step of the study, i.e., the analysis of the FAAH-coding messenger RNA, the reverse transcriptase polymerase chain reaction (RT-PCR) was performed, using total RNA isolated from human lymphocytes ($10 \times 10^6$ cells) by means of the S.N.A.P.™ kit of Invitrogen (Maccarrone et al. 1998). RT-PCR reactions were performed using 100 ng of total RNA, for the amplification of FAAH, or 0.4 ng for 18S rRNA, and the EZrTth RNA PCR kit (Perkin Elmer) as described (Maccarrone et al. 1998). Five μl of the reaction mixture were electrophoresed on a 6% polyacrylamide gel which was then dried and subjected to autoradiography (Maccarrone et al. 1998). Used primers are reported in the annexed sequence listing from SEQ ID NO: 1 to SEQ ID NO: 4

The results of the RT-PCR analysis are reported in Figure 2B, confirming that the decrease of the FAAH protein is associated to a decrease of the mRNA coding for the enzyme. Hence, in the lymphocytes of the subjects experiencing spontaneous abortion there is a reduced expression of the gene coding for the FAAH.

Then, after completing the preliminary study A, a further study (study B) was designed and performed, to ascertain whether the detected association between FAAH content and miscarriage occurrence could predict eventual abortion as well, and, in the affirmative case, to set a threshold value (1) indicative of this condition at an early stage.

Study B, conducted on <8-week-pregnant women, has clearly shown that a decreased FAAH activity and content in peripheral lymphocytes below a certain critical threshold is an early marker of spontaneous abortion, occurring about 2 weeks later.

In study B, the method of the present invention was applied to a cohort of 120 <8-week-pregnant low-risk young women (age range, 28 to 35 years). This study was performed with repeated US and with a single blood test between 7 and 8 weeks of pregnancy. The inclusion criteria were the same adopted for study A.

In the cohort, 15 asymptomatic women (i.e., subjects showing no signs of impending abortion such as vaginal bleeding or abdominal pain) had a FAAH content < 0.15 (Fig. 1C). Transvaginal echographic evaluation showed that in these 15 women spontaneous abortion occurred approximately 10 days later. The remaining 105 subjects of the cohort had FAAH levels ≥0,15 and only one woman from this "normal" group miscarried, though her clinical details could not account for this peculiarity. Statistical analysis performed by the Mann-Whitney test showed that the difference between the mean values of FAAH content in the 16 women who miscarried ($0.13 \pm 0.02$ absorbance units at 405 nm) and the 104 with normal gestation ($0.25 \pm 0.03$) was statistically significant (P < 0.0001). Also the Fisher's exact test indicated that the association between low FAAH content and spontaneous abortion was statistically significant. Therefore, the method of the present invention thus applied demonstrated the FAAH levels, measured on 20 μg of lymphocyte proteins, to be significantly lower in the 15 subjects that would have miscarried after approximately two weeks, with respect to the 105 with normal gestation (Fig. 2), thereby demonstrating the diagnostic value of this novel procedure.

These results show that a decreased activity and expression of the FAAH enzyme in peripheral lymphocytes, as well as in platelets and in general in all blood cells also according to what is known to the art (see in particular Maccarrone et al. in *FEBS Lett* 1999; Wagner et al. 1999; Maccarrone et al. 2000), is an early marker (even at an <8-week gestation stage) of miscarriage in humans (Maccarrone et al. 2000).

Peripheral lymphocytes were chosen in this investigation, in particular because these cells play a critical role in embryo implantation and successful pregnancy in humans (Redline et al. 1999; Piccinni et al. 1998). Moreover, lymphocytes are well-represented in blood and can be isolated with routine procedures.

Moreover, a crucial result in the analysis of the FAAH protein content is that a threshold value (1) of about $0.15 \pm 0.02$ could be assessed. This means that women with $1 < 0.15 \pm 0.02$ experienced a spontaneous abortion, whereas those with $1 = 0.15 \pm 0.02$ did not (FIG. 1B). Since ELISA tests are quicker and cheaper than radiochromatographic or RT-PCR analyses, they were chosen to further investigate the association between low FAAH and miscarriage.

So far only a general description of the present invention has been provided. With the aid of the following examples, which ought to be construed as merely illustrative, a more detailed description of specific embodiments thereof will hereinafter be provided, aimed at making more apparent the objects, the features, the advantages and the operation steps of the invention.

Examples

Example 1: Lymphocyte isolation from peripheral blood

5 ml peripheral blood drawn from the forearm vein of a patient were mixed in 50 ml Falcon tubes (Becton Dickinson, NJ, U.S.A.) containing a citrate-dextrose solution (Sigma Chemical Company, MO, U.S.A.) in a 6:1 blood/ solution ratio.

The mixture blood/citrate-dextrose solution was then diluted with phosphate-buffered saline (PBS,; Sigma Chemical Company, MO, U.S.A.) in a 1:1 ratio.

One volume of Lympholyte®-H (Cedarlane®, Ontario, Canada) was put in another 50 ml Falcon tube, and subsequently an equal volume of blood/citrate-dextrose solution/PBS was lightly poured thereon. The solution was then centrifuged for 20 min at 450xg in a centrifuge with no brake, at room temperature.

Lymphocytes were then recovered with a Pasteur pipette at the interface between the plasma and the Lympholyte®-H separation medium, washed in 5 ml PBS and centrifuged for 20 min at 450xg. The lymphocyte-containing pellet was then recovered, and resuspended in 200 µl PBS.

20 µl of lymphocyte suspension were then collected and diluted in 20 µl Trypan blue solution (ICN Biomedicals Inc., OH, U.S.A.) (0.4% in PBS), in order to allow lymphocyte counting in a Neubauer chamber by an optical microscope.

A suspension volume equivalent to $5 \times 10^6$ cells was then collected and used for the immunoenzymatic assay.

All the steps of the isolation procedure were performed at room temperature.

Example 2: Platelet isolation from peripheral blood

5 ml peripheral blood drawn from the forearm vein of a patient were collected in 15 ml Falcon tubes (Becton Dickinson, NJ, U.S.A.) containing a citrate-dextrose solution (Sigma Chemical Company, MO, U.S.A.) in a 6:1 blood/ solution ratio.

The resulting mixture was then centrifuged at 150xg for 15 min at room temperature, and the supernatant was collected by a plastic Pasteur pipette, carefully avoiding contact with the pellet.

The platelet-enriched supernatant was then centrifuged 15 min at 1000xg at room temperature, and the pellet was resuspended in 200 µl phosphate-buffered saline (PBS; Sigma Chemical Company, MO, U.S.A.). Different suspension volumes were then added to different Falcon plate wells, in the same concentration range used for the lymphocytes as to example 1.

Example 3: Immunoenzymatic assay by Cellular ELISA

One volume of the cellular suspension described in Example 1 was added to the wells of a 96-well Falcon plate (Becton Dickinson, NJ, U.S.A.). To each well a different volume of cellular suspension was added, up to a maximum of $1.0 \times 10^6$ lymphocytes (no more than 20 µl; in particular 0 - 2 - 4 - 8 - 12 - 16 - 20). Then, bidistilled water was added to each well, to a final volume of 180 µl. The resulting mixture was incubated 5 min at room temperature.

To each well 20 µl of a coating buffer (C.B.) (500 mM $Na_2CO_3$, 0.2% $NaN_3$, at pH 9.6) were added. The resulting mixture was incubated for 2 hours at room temperature.

The content of each well was then removed and replaced with 200 µl of a blocking buffer (B.B.) (physiological solution buffered with phosphates (PBS), 1% gelatin, 0.01% Tween-20). Then the resulting mixture was incubated for 2 hours at room temperature.

Each well was then washed 4 times with 200 µl washing buffer (W.B.) (PBS, 0.01% Tween-20) and once with 200 µl diluting buffer (D.B.) (PBS, 0.3% gelatin, 0.01% Tween-20).

100 µl of human FAAH-specific antibodies were added to each well. They were produced in the rabbit against an ovalbumin-conjugated peptide, corresponding to the conserved FAAH sequence reported in the sequence listing as SEQ ID NO:5 (anti-FAAH antibodies). These polyclonal antibodies were diluted 1:200 in D.B, and the resulting mixture was incubated for 1.5 hours at room temperature.

Each well was then washed 4 times with 200 µl W.B. and once with 200 µl D.B.

Then, 100 µl goat anti-rabbit polyclonal antibodies conjugated with alkaline phosphatase (GAR-AP, Bio-Rad Laboratories CA, U.S.A.), diluted 1:2000 in D.B., were added to each well and the resulting mixture was incubated for 1.5 hours at room temperature.

Each well was then washed 5 times with 200 µl W.B., and 150 µl of *p*-nitrophenylphosphate solution in a 0.5 M diethanolamine buffer, pH = 9.8 (0.5 mg/ml) were added thereto. All was incubated in the dark for 2 hours and at room temperature, and then 30 µl of 3M NaOH solution were added to each well. The absorbance values due to the hydrolysis of *p*-nitrophenylphosphate by alkaline phosphatase were then determined in a plate reader (e.g., "microtiter plate reader"), with a 405 nm wavelength filter. The results are reported in Figures 2B and 2C

The $Na_2CO_3$, $NaN_3$ and PBS salts, as well as the *p*-nitrophenylphosphate, the diethanolamine, the NaOH and the BSA, were purchased from Sigma Chemical Company (MO, U.S.A.), whereas the gelatin and the Tween-20, both EIA grade, were purchased from Bio-Rad Laboratories (CA, U.S.A.).

The same process was performed on a cellular suspension volume as to example 2. The results are reported in Figure 3.

Example 4: Active Anandamide Detection by HPLC

FAAH (arachidonoylethanolamide amidohydrolase, EC 3.5.1.4) activity in human lymphocyte homogenates (20 µg/test) was determined by measuring the release of [1-$^{14}$C]arachidonic acid from [1-$^{14}$C]anandamide (NEN Du-Pont de Nemours, 52 mCi/mmol), using reverse phase high performance liquid cromatography as reported (Maccarrone et al. 1998). It has to be pointed out that the use of [$^3$H]anandamide, measuring the release of [$^3$H]arachidonic acid, yields the same results.

The results are reported in Figure 1A.

Example 5: FAAH detection by Western blot

The Western blot of FAAH was performed on human lymphocyte homogenates (20 µg/sample), separated by 12% SDS polyacrilamide gel, and electroblotted on 0.45 µm nitrocellulose filters, using specific FAAH polyclonal antibodies (Primm), diluted 1:200, and then by alkaline phosphatase-conjugated goat anti-rabbit polyclonal antibodies, (Bio-Rad Laboratories), diluted 1:2000 (16) (Maccarrone et al. 1998). This Western blot has identified FAAH as a single immunoreactive band of about 67 kDa (figure 2A).

For the results see Figure 2A.

Example 6: FAAH detection by RT-PCR

A total RNA, isolated from human lymphocytes ($10X10^6$ cells) by the Invitrogen S.N.A.P.™ kit (Maccarrone et al. 1998), was used. RT-PCR reactions were carried out using 100 ng total RNA for FAAH amplification, or 0.4 ng for the 18S rRNA, using the EZrTth RNA PCR kit (Perkin Elmer) as described (Maccarrone et al., 1998). 5 µl reaction mixture were electrophoresed on a 6% polyacrylamide gel which was then dried and subjected to autoradiography (Maccarrone et al 1998). The primers used are those corresponding to the annexed sequence listing, from SEQ ID NO:1 to SEQ ID NO:4

The results are reported in Figure 2B.

References

- Calignano A, La Rana G, Giuffrida A, Piomelli D. Control of pain initiation by endogenous cannabinoids. Nature 1998; 394: 277-81.
- Cole LA, Isozaki T, Jones EE. Urine beta-core fragment, a potential screening test for ectopic pregnancy and spontaneous abortion. *Fetal Diagn Ther* 1997; 12: 336-39.
- Das SK, Paria BC, Chakraborty I, Day SK. Cannabinoid ligand-receptor signaling in the mouse uterus. *Proc Natl Acad Sci USA* 1995; 92: 4332-36.
- Devane WA, Hannus L, Breuer A, et al. Isolation and structure of a brain constituent that binds to the cannabinoid receptor. *Science* 1992; 258: 1946-49.
- Di Marzo V, Sepe N, De Petrocellis L, et al. Trick or treat from food endocannabinoids? *Nature* 1998; 396: 636.
- Giang DK, Cravatt BF. Molecular characterization of human and mouse fatty acid amide hydrolase. Proc Natl Acad Sci USA 1997; 94: 2238-42.
- Goldstein SR. Embryonic death in early pregnancy: a new look at the first trimester. *Obstet Gynecol* 1994; 84: 294-97.
- Goldstein SR. Sonography in early pregnancy failure. *Clin Obstet Gynecol* 1994; 37: 681-92.
- Kline J, Stein Z, Susser M. Conception to birth: epidemiology of prenatal development. New York: Oxford University Press, 1989.
- Kovalevskaya G, Birken S, Kakmuma T, Schlatterer J, O'Connor JF. Evaluation of nicked human chorionic gonadotropin content in clinical specimens by a specific immunometric assay. *Clin Chem* 1999; 45: 68-77.
- Maccarrone M, Bari M, Finazzi-Agrò A. A sensitive and specific radiochromatographic assay of fatty acid amide hydrolase. *Anal. Biochem.* 1999; 267, 314-318.

- Maccarrone M, Bari M, Menichelli A, Del Principe D, Finazzi Agrò A. Anandamide activates human platelets through a pathway independent of the arachidonate cascade. *FEBS Lett* 1999; 447: 277-282.
- Maccarrone M, Catani MV, Finazzi-Agrò A, Melino G. Involvement of 5-lipoxygenase in programmed cell death of cancer cells. *Cell Death Differ* 1997; 4: 396-02.
- Maccarrone M, Van der Stelt M, Rossi A, Veldink GA, Vliegenthart JFG, Finazzi-Agrò A. Anandamide hydrolysis by human cells in culture and brain. *J Biol Chem* 1998; 273: 32332-39.
- Maccarrone M., Fiorucci L., Erba F., Bari M., Finazzi Agrò A., Ascoli F., Human mast cells take up and hydrolize anandamide under the control of 5-lipoxygenase and do not express cannabinoid receptors. *FEBS Lett* 2000; 468: 176-80.
- Ness RB, Grisso JA, Hirschinger N, et al. Cocaine and tobacco use and the risk of spontaneous abortion. N *Engl J Med* 1999; 340: 333-39.
- Okon MA, Laird SM, Tuckerman EM, Li TC. Serum androgen levels in women who have recurrent miscarriages and their correlation with markers of endometrial function. *Fertil Steril* 1998; 69: 682-90.
- Paria BC, Ma W, Andrenyak DM, et al. Effects of cannabinoids on preimplantation mouse embryo development and implantation are mediated by brain-type cannabinoid receptors. *Biol Reprod* 1998; 58: 1490-95.
- Paria BC, Zhao X, Wang J, Das SK, Dey SK. Fatty-acid amide hydrolase is expressed in the mouse uterus and embryo during the periimplantation period. *Biol. Reprod.* 1999; 60: 1151-57.
- Pertwee RG. Pharmacology of cannabinoid CB$_1$ and CB$_2$ receptors. *Pharmacol Ther* 1997; 74: 129-80.
- Piccinni MP, Beloni L, Livi C, Maggi E, Scarselli G, Romagnani S. Defective production of both leukemia inhibitory factor and type 2 T-helper cytokines by decidual T cells in unexplained recurrent abortions. *Nature Med* 1998; 4: 1020-24.
- Redline RW, Zaragoza M, Hassold T. Prevalence of developmental and inflammatory lesions in nonmolar first-trimester spontaneous abortions. *Hum Pathol* 1999; 30: 93-00.
- Schmid PC, Paria BC, Krebsbach RJ, Schmid HHO, Dey SK. Changes in anandamide levels in mouse uterus are associated with uterine receptivity for embryo implantation. *Proc Natl Acad Sci USA 1997;* 94: 4188-92.
- Sozio J, Ness RB. Chlamydial lower genital tract infection and spontaneous abortion. *Infect Dis Obstet Gynecol* 1998; 6: 8-12.
- Spandidos DA, Koumantakis E, Sifakis S, Sourvinos G. Microsatellite mutations in spontaneously aborted embryos. *Fertil Steril* 1998; 70: 892-95.
- Stella N, Schweitzer P, Piomelli D. A second endogenous cannabinoid that modulates long-term potentiation. *Nature* 1997; 388: 773-78.
- Wagner J.A., Varga K., Jarai Z. and Kunos G., Mesenteric vasodilation mediated by endothelial anandamide receptors. *Hypertension* 1999; 33, 429-434.
- Wang X, Wang M, Niu T, Chen C, Xu X. Microsomal epoxide hydrolase polymorphism and risk of spontaneous abortion. *Epidemiology* 1998; 9: 540-44.
- Wilson K e Walker J. Metodologia biochimica. 1995. Raffaello Cortina Editore, Milano.
- Yang ZM, Paria BC, Dey SK. Activation of brain-type cannabinoid receptors interferes with preimplantation mouse embryo development. *Biol Reprod* 1996; 55: 756-61.

SEQUENCE LISTING

GENERAL INFORMATION:

(i) APPLICANT: Università degli Studi di Roma "Tor Vergata"

(ii) TITLE OF INVENTION:

METHOD AND KIT FOR THE EARLY DIAGNOSIS OF MISCARRIAGE IN HUMAN BEINGS

(iii)     NUMBER OF SEQUENCES: 5

(iv)     CORRESPONDENCE ADDRESS:

      (A) ADDRESSEE: Società Italiana Brevetti

      (B) STREET: Piazza di Pietra, 39

      (C) CITY: Rome

      (D) COUNTRY: Italy

      (E) POSTCODE: I-00186 .

(v)  COMPUTER READABLE FORM:

      (A) MEDIUM TYPE:

      (B) COMPUTER: IBM compatible PC

      (C) OPERATING SYSTEM: PC-DOS/MS-DOS Rev. 5.0

      (D) SOFTWARE: Microsoft Word 97

(viii)     ATTORNEY INFORMATION

          (A) NAME: Bazzichelli, Alfredo (Eng.)

          (B) REFERENCE: RM/X89582/EP-EBR

(ix)     TELECOMMUNICATION INFORMATION

          (A) TELEPHONE: 06/695441

          (B) TELEFAX:  06/69444830

          (C) TELEX:    612287 ROPAT

(1) INFORMATION FOR SEQ SEQ ID NO: 1:

(i) SEQUENCE CHARACTERISTICS

      (A) LENGTH: 22 bp

      (B) TYPE: nucleic acid

      (C) STRANDEDNESS: single

      (D) TOPOLOGY: linear

(ii) MOLECULE TYPE: oligonucleotide

(ix) FEATURES

      (D) OTHER INFORMATION: primer for RT-PCR on mRNA coding for the enzyme anandamide hydrolase

(xi) SEQUENCE DESCRIPTION: SEQ ID NO: 1:

TGGAAGTCCT CCAAAAGCCC AG                                    22


(2)  INFORMATION FOR SEQ ID NO: 2:

(i)  SEQUENCE CHARACTERISTICS

    (A)  LENGTH: 23 bp

    (B)  TYPE: nucleic acid

    (C)  STRANDEDNESS: single

    (D)  TOPOLOGY: linear

(ii) MOLECULE TYPE: oligonucleotide

(ix) FEATURES

    (D)  OTHER INFORMATION: primer for RT-PCR on mRNA
coding for the enzyme anandamide hydrolase

(xi) SEQUENCE DESCRIPTION: SEQ ID NO: 2:

TGTCCATAGA CACAGCCCTT CAG                                   23

(3)  INFORMATION FOR SEQ SEQ ID NO: 3:

(i)  SEQUENCE CHARACTERISTICS

    (A)  LENGTH: 20 bp

    (B)  TYPE: nucleic acid

    (C)  STRANDEDNESS: single

    (D)  TOPOLOGY: linear

(ii) MOLECULE TYPE: oligonucleotide

(ix) FEATURES

    (D)  OTHER INFORMATION: primer for RT-PCR on
ribosomal RNA 18S used as control

(xi) SEQUENCE DESCRIPTION: SEQ ID NO: 3:

AGTTGCTGCA GTTAAAAAGC                                       20


(4)  INFORMATION FOR SEQ SEQ ID NO: 4:

(i)  SEQUENCE CHARACTERISTICS

    (A)  LENGTH: 20 bp

    (B)  TYPE: nucleic acid

    (C)  STRANDEDNESS: single

    (D)  TOPOLOGY: linear

(ii) MOLECULE TYPE: oligonucleotide

(ix) FEATURES

    (D)  OTHER INFORMATION: primer for RT-PCR on
ribosomal RNA 18S used as control

(xi) SEQUENCE DESCRIPTION: SEQ ID NO: 4:

CCTCAGTTCC GAAAACCAAC                                                      20

(5)  INFORMATION FOR SEQ SEQ ID NO: 5:

(i)  SEQUENCE CHARACTERISTICS

    (A)  LENGTH: 17 amino acids

    (B)  TIPO: amino acidic

    (C)  STRANDEDNESS: single

    (D)  TOPOLOGY: linear

(ii)  MOLECULE TYPE: peptide

(ix)  FEATURES

    (D)  OTHER  INFORMATION:  sequence  derived  from  the protein anadamide hydrolase used as target for polyclonal anti-anandamide hydrolase antibodies

(xi) SEQUENCE DESCRIPTION: SEQ ID NO: 5:

Val Gly Tyr Tyr Glu Thr Asp Asn Tyr Thr Met Pro Ser Pro  14

Ala Met Arg                                                               17

Annex to the application documents - subsquently filed sequences listing

```
                            SEQUENCE LISTING


        <110> Università degli Studi di Roma "Tor Vergata"


        <120> METHOD AND KIT FOR THE EARLY DIAGNOSIS OF MISCARRIAGE
              IN HUMAN BEINGS


        <130> RM/X89582/EP-EBR/mp


        <140> 00830285.3
        <141> 2000-04-14


        <160> 5


        <170> PatentIn Ver. 2.1


        <210> 1
        <211> 22
        <212> DNA
        <213> Artificial Sequence


        <220>
        <223> Description of Artificial Sequence: synthetic
              oligonucleotide


        <220>
        <223> primer for RT-PCR on mRNA coding for the enzyme
              anandamide hydrolase


        <400> 1
        tggaagtcct ccaaaagccc ag                                     22



        <210> 2
        <211> 23
        <212> DNA
        <213> Artificial Sequence


        <220>
        <223> Description of Artificial Sequence: synthetic
              oligonucleotide


        <220>
        <223> primer for RT-PCR on mRNA coding for the enzyme
              anandamide hydrolase


        <400> 2
        tgtccataga cacagccctt cag                                    23
```

```
<210> 3
<211> 20
<212> DNA
<213> Artificial Sequence


<220>
<223> Description of Artificial Sequence: synthetic
      oligonucleotide


<220>
<223> primer for RT-PCR on ribosomal RNA 18S used as a
      control


<400> 3
agttgctgca gttaaaaagc                                              20



<210> 4
<211> 20
<212> DNA
<213> Artificial Sequence


<220>
<223> Description of Artificial Sequence: synthetic
      oligonucleotide


<220>
<223> primer for RT-PCR on ribosomal RNA 18S used as
      control


<400> 4
cctcagttcc gaaaaccaac                                              20



<210> 5
<211> 17
<212> PRT
<213> Artificial Sequence


<220>
<223> Description of Artificial Sequence: synthetic
      peptide


<220>
<223> sequence derived from the protein anandamide
      hydrolase used as target for polyclonal
```

anti-anandamide hydrolase antibodies

<400> 5

Val Gly Tyr Tyr Glu Thr Asp Asn Tyr Thr Met Pro Ser Pro Ala Met
 1               5                   10                  15

Arg

**Claims**

1.  A method for the diagnosis of spontaneous abortion in female human beings comprising the following steps:

    a. detecting the presence of the enzyme anandamide hydrolase (FAAH) active in the blood cells of said female human being, said detection yielding a detected value;
    b. comparing said detected value obtained in step a., to a threshold value, said threshold value being predetermined in order to be indicative of the probability of spontaneous abortion in female human beings.

2.  The method according to claim 1, wherein said detection is performed by an assay of the activity of said enzyme.

3.  The method according to claim 2, wherein said assay of the activity of said enzyme is performed by HPLC.

4.  The method according to claim 3, wherein the enzyme-substrate reaction is performed with a 20µg/200 µl ratio between the protein amount and the enzymatic reaction mixture volume, and the subsequent extracting step is performed with a 1:4 volume ratio between the reaction mixture and the extraction mixture.

5.  The method according to claim 3, wherein said reaction between the enzyme and the substrate is conducted in a C18 column having dimensions 5 µm, 30 x 3 mm.

6.  The method according to claim 1, wherein said detection is performed by measuring the expression of said enzyme in said cells.

7.  The method according to claim 6, wherein said measuring of the expression of said enzyme in said cells is performed by measuring the presence of the messenger RNA coding for said enzyme in said cells.

8.  The method according to claim 7, wherein said measuring of the expression of said enzyme is performed by reverse transcriptase polymerase chain reaction (RT-PCR).

9.  The method according to claim 5, wherein said measuring of the expression of said enzyme is performed by measuring the presence of said enzyme in said cells.

10. The method according to claim 9, wherein said measuring of the presence of said enzyme is performed by ELISA.

11. The method according to claim 10, wherein the cell homogenating step is replaced by the step of lysing said cells on the medium onto which the binding reaction between the enzyme and the specific antibody takes place.

12. The method according to claim 10 or 11, wherein said measuring is performed using the following compositions:

    a coating composition comprising a chemical reactant able to adhere the cell proteins onto the well walls and an agent inhibiting the growth of microorganisms at the reaction temperature;
    a washing composition comprising a physiological buffered solution and a detergent;
    a blocking composition comprising the washing composition and a saturating protein, said saturating protein being a protein able to saturate the aspecific sites of the reaction vessel, said protein being comprised in a sufficient amount in order to attain, following the addition of said blocking composition in said reaction vessel, the saturation of said aspecific sites of said reaction vessel; and
    a diluting composition comprising the washing composition and the saturating protein comprised in the blocking composition, said saturating protein being comprised in said diluting composition in an amount equal to one third of the amount of said saturating protein in the blocking composition;
    by the following steps in sequence:

    -   adding to a reaction vessel a different cell suspension volume equal to 1/10 of the final volume;
    -   adding to said vessel bidistilled water or equivalent solution to a volume equal to 8/10 of the final volume, and incubating it for ≤ 5 min at room temperature;
    -   adding to said vessel a volume equal to 1/10 of the final volume of the coating composition, and incubating all for ≤ 2 hours at room temperature;
    -   collecting the content of said vessel and adding an amount of blocking composition sufficient to fill said vessel, and incubating all for ≤ 2 hours at room temperature;

- washing said vessel at least 4 times with the washing composition, and once with the diluting composition;
- adding to said vessel a volume equal to one half of the final volume of the diluting composition and a primary anandamide hydrolase-specific antibody suitably diluted therein in order to obtain an effective bond to the target enzyme and incubating all for ≤ 1.5 hours at room temperature;
- washing said well at least 4 times with the washing composition, and once with the diluting composition;
- adding to each well a volume equal to one half of the final volume of a secondary antibody specific for said primary antibody suitably diluted in the diluting composition in order to allow the measurable bond between said secondary antibody and the primary antibody, and incubating all for ≤ 1.5 hours at room temperature;
- washing said well at least 5 times with one volume of the washing composition and adding the substrate of the enzyme conjugated to said secondary antibody, in an amount thereof sufficient to allow the measuring of the reaction;
- incubating, under conditions adequate to allow the completion of the reaction between the enzyme conjugated to said secondary antibody and the related substrate.

13. The method according to claim 12, wherein

the chemical reactant and the agent inhibiting the growth of microorganisms in said coating composition are 500 mM $Na_2CO_3$ and 0.2% $NaN_3$, respectively;
the physiological buffered solution and the detergent in said washing composition are a physiological buffered solution and 0.01% Tween-20, respectively; and
the saturating protein in said blocking composition and in said diluting composition is gelatin.

14. The method according to any one of the claims 1 to 13, wherein the step as to a. is preceded by the blood cells isolating step.

15. The method according to any one of the claims 1 to 14, wherein said blood cells are lymphocytes.

16. A kit for the diagnose of miscarriage comprising the following compositions:

a coating composition comprising a chemical reactant able to adhere the cell proteins onto the well walls and an agent inhibiting the growth of microorganisms at the reaction temperature;
a primary antibody composition comprising a primary antibody, said primary antibody being a specific anti-anandamide hydrolase (FAAH) antibody and a compatible carrier,
wherein said compositions are useful in the method according to any one of the claims 9 to 15.

17. The kit according to claim 16, wherein the chemical reactant and the agent of the coating composition are 500 mM $Na_2CO_3$ and 0.2% $NaN_3$ respectively, and the pH of said composition is 9.6.

18. The kit according to claim 16 or 17, wherein said kit further comprises a washing composition comprising a physiological buffered solution and a detergent, said washing composition being useful in the method according to any one of the claims 9 to 15.

19. The kit according to claim 18, wherein said physiological buffered solution is buffered with a phosphate buffer (PBS) and said detergent is 0.01% Tween-20.

20. The kit according to any one of the claims 16 to 19, further comprising

a blocking composition comprising the washing composition as defined in claims 16 to 19 and a saturating protein, said saturating protein being a protein able to saturate the aspecific sites of the reaction vessel, said saturating protein being comprised in said blocking composition in an amount sufficient to obtain, following the addition of said blocking composition in said reaction vessel, the saturation of the aspecific sites of said reaction vessel; and
a diluting composition comprising the washing composition as defined in claims 16 to 19 and the saturating protein comprised in the blocking composition, said saturating protein being comprised in said diluting composition in an amount equal to one third of the amount of said saturating protein in the blocking composition, wherein said blocking composition and said diluting composition are useful in the method according to any one of the claims 9 to 15.

**21.** The kit according to claim 20, wherein said saturating protein is gelatin.

**22.** The kit according to any one of the claims 16 to 19, wherein said kit further comprises
a blocking protein composition comprising, in a compatible carrier, the saturating protein as defined in claims 20 or 21, in such an amount that, following the addition of said blocking protein composition to an adequate quantity of washing composition as defined in claims 16 to 19, the blocking composition as defined in claim 20 or 21 is obtained.

**23.** The kit according to any one of the claims 16 to 19, or according to claim 22, wherein said kit further comprises
a diluting protein composition comprising, in a compatible carrier, the saturating protein as defined in claim 20 or 21, in such an amount that, following the addition of said diluting protein composition to an adequate amount of washing composition as defined in claims 16 to 19, the diluting composition as defined in claim 20 or 21 is obtained.

**24.** The kit according to any one of the claims 16 to 19, further comprising a vessel for the blocking composition comprising an amount of the washing composition as defined in claims 16 to 19 such that, following the addition in said washing composition of the protein blocking composition as to claim 22 the blocking composition as defined in claim 20 or 21 is obtained.

**25.** The kit according to any one of the claims 16 to 19, or according to claim 24, further comprising a vessel for the diluting composition comprising an amount of washing composition as defined in claims 16 to 19, such that following the addition of the protein diluting composition as to claim 23, the diluting composition as defined in claim 20 or 21 is obtained.

**26.** The kit according to any one of the claims 16 to 25, wherein a vessel comprising bidistilled water is further comprised.

**27.** The kit according to any one of the claims 16 to 26, further comprising:

a secondary antibody composition, comprising, in a compatible carrier, a secondary antibody specific for the primary antibody according to claim 16, said secondary antibody being conjugated to a quantitatively detectable molecule, and
a detecting composition comprising, in a compatible carrier, a molecule that, interacting with the detectable molecule of the antibody composition, yields a measurable product.

**28.** The kit according to claim 27, wherein said detectable protein conjugated to said secondary antibody is the enzyme alkaline phosphatase, and said molecule interacting with said detectable molecule is the p-nitrophenilphosphate substrate.

**29.** The kit according to any one of the claims 16 to 28, wherein the compositions are 10X concentrated.

**30.** The kit according to any one of the claims 16 to 29, further comprising a medium for conducting the ELISA reaction.

FIG.1A

FIG.1B

FIG.1C

**FIG.2**

**FIG.3**

**FIG.4**

**European Patent Office**

## EUROPEAN SEARCH REPORT

Application Number

EP 00 83 0285

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | DATABASE BIOSIS 'Online! BIOSCIENCES INFORMATION SERVICE, PHILADELPHIA, PA, US; AN-PREV200000266162, M. MACCARRONE ET AL.: "Down-regulation of anandamide hydrolase in mouse uterus by sex hormones" XP002154975 * abstract * & EUROPEAN JOURNAL OF BIOCHEMISTRY, vol. 267, May 2000 (2000-05), pages 2991-2997, | 1-10,12, 16 | C12Q1/34 C12Q1/68 G01N33/573 |
| Y | B. C. PARIA ET AL.: "The preimplantation mouse embryo is a target for cannabinoid ligand-receptor signaling" PROCEEDINGS OF THE NATIONAL ACADEMY OF SCIENCES OF USA, vol. 92, October 1995 (1995-10), pages 9460-9464, XP002154971 WASHINGTON US * abstract * | 1,6-9 | |
| D,Y | P. C. SCHMID ET AL.: "Changes in anandamide levels in mouse uterus are associated with uterine receptivity for embryo implantation" PROCEEDINGS OF THE NATIONAL ACADEMY OF SCIENCES OF USA, vol. 94, April 1997 (1997-04), pages 4188-4192, XP002154972 WASHINGTON US * abstract *<br><br>-/-- | 1 | TECHNICAL FIELDS SEARCHED (Int.Cl.7)<br><br>C12Q<br>G01N |

The present search report has been drawn up for all claims

| Place of search | Date of completion of the search | Examiner |
|---|---|---|
| THE HAGUE | 8 December 2000 | Griffith, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 146 128 A1

**European Patent Office**

## EUROPEAN SEARCH REPORT

Application Number

EP 00 83 0285

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | M. MACCARRONE ET AL.: "A sensitive and specific radiochromatographic assay of fatty acid amide hydrolase activity" ANALYTICAL BIOCHEMISTRY, vol. 267, 1999, pages 314-318, XP002154973 NEW YORK US * the whole document * | 1-5 | |
| Y | I. MATSUNAGA ET AL.: "Further characterization of hydrogen peroxide-dependent fatty acid alpha-hydroxylase from Sphingomonas paucimobilis" JOURNAL OF BIOCHEMISTRY., vol. 124, no. 1, July 1998 (1998-07), pages 105-110, XP002154974 JAPANESE BIOCHEMICAL SOCIETY, TOKYO., JP ISSN: 0021-924X * page 105 - page 106 * | 1,2,10, 12,16 | |
| A | US 5 925 672 A (D. PIOMELLI ET AL.) 20 July 1999 (1999-07-20) * examples * | | TECHNICAL FIELDS SEARCHED (Int.Cl.7) |
| A | DATABASE BIOSIS 'Online! BIOSCIENCES INFORMATION SERVICE, PHILADELPHIA, PA, US; AN-PREV199900260342, B. C. PARIA ET AL.: "Fatty-acid amide hydrolase is expressed in the mouse uterus and embryo durin the periimplantation period" XP002154976 * abstract * | | |
| D | & BIOLOGY OF REPRODUCTION, vol. 60, 1999, pages 1151-1157, | | |

The present search report has been drawn up for all claims

| Place of search | Date of completion of the search | Examiner |
|---|---|---|
| THE HAGUE | 8 December 2000 | Griffith, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

27

**ANNEX TO THE EUROPEAN SEARCH REPORT
ON EUROPEAN PATENT APPLICATION NO.**

EP 00 83 0285

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2000

| Patent document cited in search report | Publication date | Patent family member(s) | Publication date |
|---|---|---|---|
| US 5925672 A | 20-07-1999 | AU 7622198 A<br>EP 0954303 A<br>WO 9824396 A | 29-06-1998<br>10-11-1999<br>11-06-1998 |

EPO FORM P0459

For more details about this annex : see Official Journal of the European Patent Office, No. 12/82